# FASCICULE DE BREVET EUROPEEN

(11) **EP 1 773 783 B1**
(45) Date de publication et mention de la délivrance du brevet: **05.05.2010**
(21) Numéro de dépôt: 05790868.3
(22) Date de dépôt: 20.07.2005
(51) Int. Cl.: C07D 231/14

(54) **PROCEDE DE PREPARATION DE DERIVES N-PIPERIDINO-1,5-DIPHENYLPYRAZOLE-3-CARBOXAMIDE**
VERFAHREN ZUR HERSTELLUNG VON N-PIPERIDINO-1,5-DIPHENYLPYRAZOL-3-CARBOXAMID-DERIVATEN
METHOD FOR PREPARING N-PIPERIDINO-1,5-DIPHENYLPYRAZOLE-3-CARBOXAMIDE DERIVATIVES

(30) Priorité: 22.07.2004 FR 0408111
(43) Date de publication de la demande: 18.04.2007
(73) Titulaire: Sanofi-Aventis, 75013 Paris (FR)
(72) Inventeur: DAUMAS, Marc, F-04700 Oraison (FR); DLUBALA, Alain, F-30133 Les Angles (FR); SOLE, Raphaël, F-30300 Comps (FR); VAYRON, Philippe, FR-05300 Chateauneuf de Chabre (FR)
(74) Mandataire: Kugel, Dominique
(86) Numéro de dépôt international: PCT/FR2005/001850
(87) Numéro de publication internationale: WO 2006/021652

(56) Documents cités:
- EP-A- 0 656 354

## Description

La présente invention a pour objet un procédé de préparation d'un composé de formule : 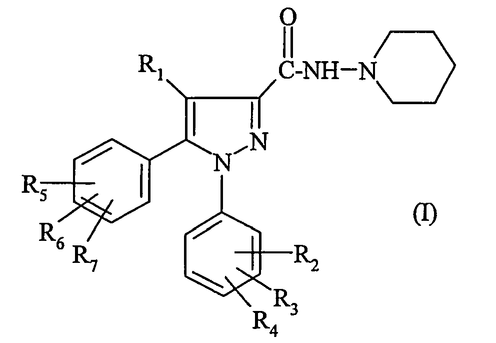 dans laquelle :
- R₁ représente un atome d'hydrogène ou d'halogène ou un groupe (C₁-C₄)alkyle ;
- R₂, R₃, R₄, R₅, R₆, R₇ représentent chacun indépendamment l'un de l'autre un atome d'hydrogène ou d'halogène ou un groupe (C₁-C₄)alkyle, (C₁-C₄)alcoxy, trifluorométhyle ;
   ainsi que de ses sels.

Les composés de formule (I) sont décrits dans différents brevets ou demandes de brevets, EP 0 656 354 B, EP 150 961 B, comme antagonistes des récepteurs CB₁ aux cannabinoïdes. Plus particulièrement le N-pipéridino-5-(4-chlorophényl)-1-(2,4-dichlorophényl)-4-méthylpyrazole-3-carboxamide ou rimonabant a montré une activité en clinique dans l'obésité et le sevrage tabagique.

Dans l'art antérieur, il est connu de préparer un composé de formule (I) par action de la N-aminopipéridine sur un dérivé fonctionnel de l'acide :

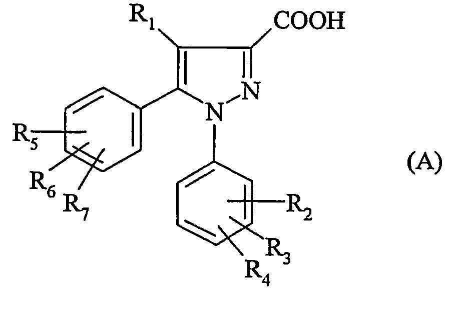

Ce procédé de synthèse est notamment décrit dans le brevet EP 0 656 354 B pour la préparation du rimonabant. Comme dérivé fonctionnel, on utilise par exemple un ester alkylique ou le chlorure d'acide.

Selon la présente invention, on prépare un composé de formule (I) par un procédé caractérisé en ce que on fait réagir un dérivé du pentane de formule X-(CH₂)₅-X' (II) dans laquelle X et X' représentent chacun indépendamment un atome d'halogène ou un groupe YSO₂O- dans lequel Y représente un groupe (C₁-C₄)alkyle, (C₁-C₄)perfluoroalkyle, phényle non substitué ou substitué par un groupe méthyle, chloro ou nitro, sur un dérivé de pyrazole-3-carbohydrazide de formule : 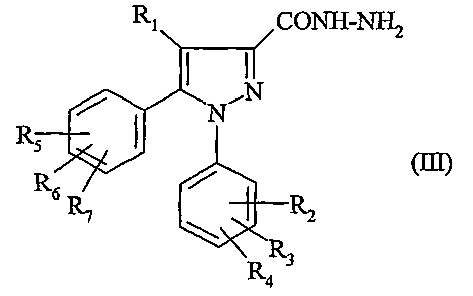 dans laquelle R₁, R₂, R₃, R_{4,} R₅, R₆, R₇ sont tels que définis ci-dessus pour (I).

La réaction est effectuée en présence d'une base dans un solvant et à une température comprise entre la température ambiante et la température de reflux du solvant.

Particulièrement, dans le procédé selon l'invention, on utilise un composé de formule (II) dans laquelle X et X' représentent chacun indépendamment un atome d'halogène.

Particulièrement aussi, on utilise un composé de formule (II) dans laquelle X et X' représentent chacun indépendamment un groupe YSO₂O- dans lequel Y est tel que défini ci-dessus.

Un mode de réalisation particulier de la présente invention est caractérisé en ce que l'on prépare le rimonabant par action d'un composé de formule (II) sur le 5-(4-chlorophényl)-1-(2,4-dichlorophényl)-4-méthyl-1*H*-pyrazole-3-carbohydrazide de formule :

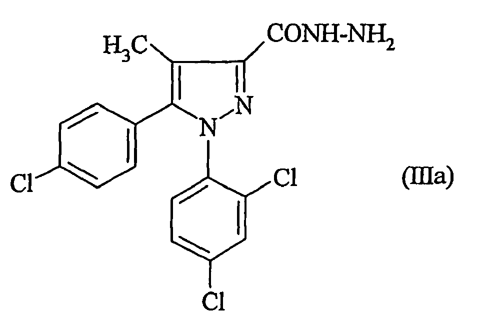

Selon un mode de réalisation particulier, on fait réagir le 1,5-dihalogénopentane de formule (II) sur le 5-(4-chlorophényl)-1-(2,4-dichlorophényl)-4-méthyl-1*H-*pyrazole-3-carbohydrazide (IIIa).

Plus particulièrement, on fait réagir le 1,5-dibromopentane sur le 5-(4-chlorophényl)-1-(2,4-dichlorophényl)-4-méthyl-1*H*-pyrazole-3-carbohydrazide (IIIa).

La réaction est réalisée en présence d'une base organique, telle qu'une amine tertiaire, par exemple la triéthylamine, ou d'une base minérale, telle que NaOH, KOH, K₂CO₃, Na₂CO₃, Cs₂CO₃.

La réaction est effectuée dans un solvant aromatique, par exemple le toluène ou le chlorobenzène, dans un solvant éthéré, par exemple le tétrahydrofurane ou le diméthoxyéthane ou dans le dioxane, ou dans un solvant nitrile, tel que l'acétonitrile ou le propionitrile.

Préférentiellement, la réaction est effectuée dans l'acétonitrile, en présence de triéthylamine ou Na₂CO₃ ou K₂CO₃.

De façon tout particulièrement préférée, la réaction est effectuée dans l'acétonitrile chauffé à reflux en présence de Na₂CO₃.

La présente invention a tout particulièrement pour objet un procédé de préparation du N-pipéridino-5-(4-chlorophényl)-1-(2,4-dichlorophényl)-4-méthylpyrazole-3-carboxamide et de ses sels, caractérisé en ce que on fait réagir le 1,5-dibromopentane avec un composé de formule (IIIa), en présence de Na₂CO₃ dans l'acétonitrile chauffé à reflux.

Par atome d'halogène on entend un atome de brome, de chlore ou d'iode.

Des composés de formule (III) et leur préparation sont connus de l'art antérieur : Canadian J. Chem. 1963, 41(7), 1813-1818 ; J. Chem. Engineering Data, 1977, 22(1), 104-110 ; J. Med. Chem., 2002, 45, 2708-2719.

La publication de J. Med. Chem., 2002, décrit en particulier le 5-(4-chlorophényl)-1-(2,4-dichlorophényl)-4-méthyl-1*H*-pyrazole-3-carbohydrazide et sa préparation à partir du chlorure d'acide correspondant.

Les exemples suivants illustrent l'invention sans toutefois la limiter.

Dans ces exemples et dans la description, les abréviations suivantes sont utilisées:
DCM : dichlorométhane.

Les spectres de masse sont mesurés en mode d'ionisation dit Electrospray (ES).

Les spectres de résonance magnétique nucléaire du proton (RMN¹H) sont enregistrés à 200 MHz ou à 300 MHz dans le DMSO-d₆ ou dans CDCl₃. Les déplacements chimiques δ sont exprimés en parties par million (ppm).

Les signaux observés en RMN sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

### Préparation 1

### 5-(4-Chlorophényl)-1-(2,4-dichlorophényl)-4-méthyl-1H-pyrazole-3-carbohydrazide.

On place sous azote 20 g de chlorure de l'acide 5-(4-chlorophényl)-1-(2,4-dichlorophényl)-4-méthylpyrazole-3-carboxylique dans 100 ml d'éthanol et on chauffe à reflux pendant 2 heures. On laisse revenir la température à 20-25°C puis on ajoute 50 g d'hydrate d'hydrazine et on chauffe à nouveau à reflux pendant 3 heures et demie. Le milieu réactionnel est filtré à chaud puis l'éthanol est évaporé. Le milieu réactionnel est concentré puis repris par 150 ml de DCM et décanté. La phase aqueuse est éliminée, la phase organique est lavée 2 fois par 100 ml d'eau puis le DCM est évaporé. Après séchage sous vide, on obtient 16,9 g du composé attendu.
ES⁺ : [M+Na]⁺= 417,419,421,423
ES⁻ : [M-H]⁻= 393, 395, 397, 399
RMN (CDCl₃ 1H à 300 MHz) : 2.35 ppm : s : 3H ; 4,0 ppm : d : 2H ; 7,04 ppm : m:2H;7.25ppm:bm:4H;7,41ppm:d:1H;8.04ppm:m:1H.

### EXEMPLE 1

### N-Pipéridino-5-(4-chlorophényl)-1-(2,4-dichlorophényl)-4-méthylpyrazole-3-carboxamide.

On place sous azote 10 g de composé obtenu à la Préparation 1 et 5,3 g de carbonate de sodium dans 100 ml d'acétonitrile. On chauffe le milieu réactionnel à reflux de l'acétonitrile et l'on ajoute un total de 6,9 ml de 1,5-dibromopentane puis on maintient le chauffage à reflux pendant 45 heures. Le milieu est hydrolysé par addition de 50 ml d'eau, on laisse décanter puis on élimine la phase aqueuse. La phase organique est lavée 2 fois par 50 ml d'une solution aqueuse saturée de NaCl. L'acétonitrile est évaporé à sec et l'on obtient 18,4 g de produit brut. Le brut obtenu est chromatographié sur gel de silice (éluant cyclohexane/acétone : 75/25 ; v/v). On reprend le produit obtenu dans 100 ml de méthylcyclohexane et on le recritallise. On obtient 5,7 g du composé attendu pur.

RMN (DMSO-d6 1H à 200 MHz) : 1.31 ppm : bm : 2H ; 1.55 ppm : bm : 4H ; 2.22 ppm : s : 3H ; 4,0 ppm : d : 2H ; 7,22 ppm : d : 2H ; 7.43 ppm : d : 2H ; 7.56 ppm : dd : 1H ; 7.71 ppm : d : 1H ; 7.76 ppm : d : 1H ; 9.02 ppm : s : 1H.

## Revendications

1. Procédé de préparation d'un composé de formule : 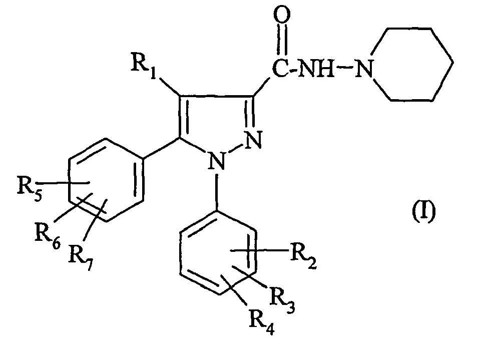 dans laquelle :
- R₁ représente un atome d'hydrogène ou d'halogène ou un groupe (C₁-C₄)alkyle;
- R₂, R₃, R₄, R₅, R₆, R₇ représentent chacun indépendamment l'un de l'autre un atome d'hydrogène ou d'halogène ou un groupe (C₁-C₄)alkyle, (C₁-C₄)alcoxy, trifluorométhyle ;
et de ses sels, **caractérisé en ce que** :
on fait réagir un dérivé du pentane de formule :
X-(CH₂)₅-X' (II)
dans laquelle X et X' représentent chacun indépendamment un atome d'halogène ou un groupe YSO₂O- dans lequel Y représente un groupe (C₁-C₄)alkyle, (C₁-C₄)perfluoroalkyle, phényle non substitué ou substitué par un groupe méthyle, chloro ou nitro, sur un dérivé de pyrazole-3-carbohydrazide de formule : 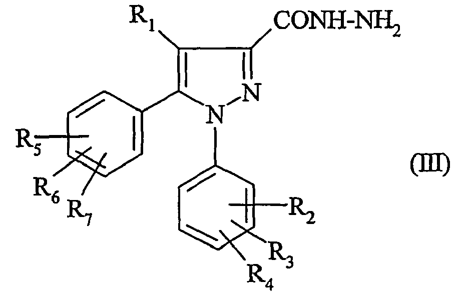 dans laquelle R₁, R₂, R₃, R₄, R₅, R₆, R₇ sont tels que définis ci-dessus pour (I) en présence d'une base, dans un solvant et à une température comprise entre la température ambiante et la température de reflux du solvant.

2. Procédé selon la revendication 1 **caractérisé en ce que** dans le composé de formule (II) X et X' représentent chacun indépendamment un atome d'halogène.

3. Procédé selon la revendication 1 **caractérisé en ce que** dans le composé de formule (II) X et X' représentent chacun indépendamment un groupe Y-SO₂-O-dans lequel Y est tel que défini dans la revendication 1.

4. Procédé selon l'une quelconque des revendications 1 à 3 pour la préparation du N-pipéridino-5-(4-chlorophényl)-1-(2,4-dichlorophényl)-4-méthylpyrazole-3-carboxamide et de ses sels, **caractérisé en ce que** :
on fait réagir un composé de formule (II) avec le 5-(4-chlorophényl)-1-(2,4-dichlorophényl)-4-méthyl-1*H*-pyrazole-3-carbohydrazide de formule :

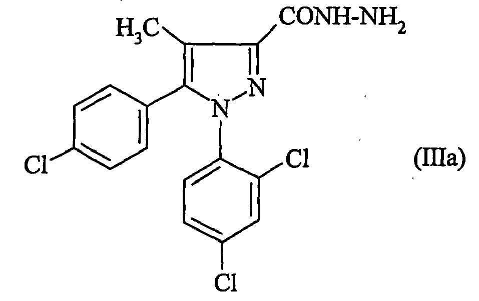

5. Procédé selon la revendication 4 **caractérisé en ce que** l'on fait réagir un 1,5-dihalogénopentane sur le composé de formule (IIIa).

6. Procédé selon la revendication 5 **caractérisé en ce que** l'on fait réagir un 1,5-dibromopentane sur le composé de formule (IIIa).

7. Procédé selon l'une quelconque des revendications 1 à 6 **caractérisé en ce que** la base est choisie parmi la triéthylamine, NaOH, KOH, K₂CO₃, Na₂CO₃, CS₂CO₃.

8. Procédé selon l'une quelconque des revendications 1 à 7 **caractérisé en ce que** le solvant est choisie parmi le toluène, le chlorobenzène, le tétrahydrofurane, le diméthoxyéthane, le dioxane, l'acétonitrile ou le propionitrile.

9. Procédé selon l'une quelconque des revendications 1 à 8 **caractérisé en ce que** la réaction est effectuée en présence de triéthylamine, de Na₂CO₃ ou de K₂CO₃ dans l'acétonitrile.

10. Procédé selon l'une quelconque des revendications 1 à 9 **caractérisé en ce que** la réaction est effectuée en présence de Na₂CO₃ dans l'acétonitrile chauffé à reflux.

11. Procédé de préparation selon la revendication 1, de N-pipéridino-5-(4-chlorophényl)-1-(2,4-dichlorophényl)-4-méthylpyrazole-3-carboxamide et de ses sels, **caractérisé en ce que** on fait réagir le 1,5-dibromopentane avec un composé de formule : 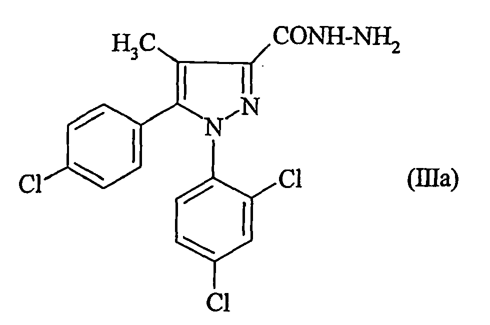 en présence de Na₂CO₃ dans l'acétonitrile chauffé à reflux.

## Claims

1. Process for the preparation of a compound of formula: 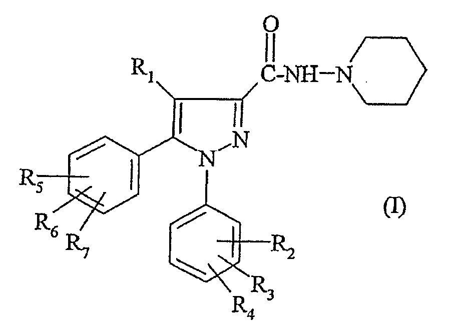 in which:
- R₁ represents a hydrogen or halogen atom or a (C₁-C₄) alkyl group;
- R₂, R₃, R₄, R₅, R₆ and R₇ represent, each independently of one another, a hydrogen or halogen atom or a (C₁-C₄) alkyl, (C₁-C₄) alkoxy or trifluoromethyl group;
and of its salts, **characterized in that**:
a pentane derivative of formula:
X- (CH₂) ₅-X' (II)
in which X and X' each independently represent a halogen atom or a YSO₂O- group in which Y represents a (C₁-C₄) alkyl group, a (C₁-C₄)perfluoroalkyl group, an unsubstituted phenyl group or a phenyl group substituted by a methyl, chloro or nitro group, is reacted with a pyrazole-3-carbohydrazide derivative of formula: 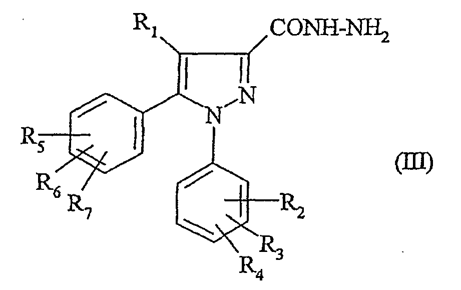 in which R₁, R₂, R₃, R₄, R₅, R₆ and R₇ are as defined above for (I), in the presence of a base, in a solvent and at a temperature between ambient temperature and the reflux temperature of the solvent.

2. Process according to Claim 1, **characterized in that**, in the compound of formula (II), X and X' each independently represent a halogen atom.

3. Process according to Claim 1, **characterized in that**, in the compound of formula (II), X and X' each independently represent a Y-SO₂-O- group in which Y is as defined in Claim 1.

4. Process according to any one of Claims 1 to 3 for the preparation of N-piperidino-5-(4-chlorophenyl)-1-(2,4-dichlorophenyl)-4-methylpyrazole-3-carboxamide and of its salts, **characterized in that**:
a compound of formula (II) is reacted with 5-(4-chlorophenyl)-1-(2,4-dichlorophenyl)-4-methyl-1*H-*pyrazole-3-carbohydrazide of formula:

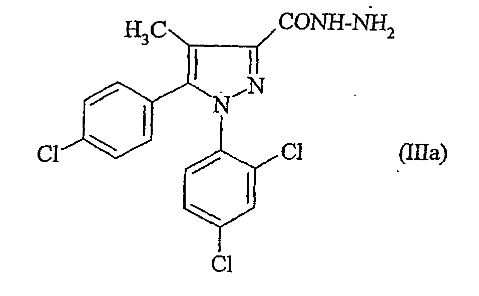

5. Process according to Claim 4, **characterized in that** a 1,5-dihalopentane is reacted with the compound of formula (IIIa).

6. Process according to Claim 5, **characterized in that** 1,5-dibromopentane is reacted with the compound of formula (IIIa).

7. Process according to any one of Claims 1 to 6, **characterized in that** the base is chosen from triethylamine, NaOH, KOH, K₂CO₃, Na₂CO₃ or CS₂CO₃ -

8. Process according to any one of Claims 1 to 7, **characterized in that** the solvent is chosen from toluene, chlorobenzene, tetrahydrofuran, dimethoxyethane, dioxane, acetonitrile or propionitrile.

9. Process according to any one of Claims 1 to 8, **characterized in that** the reaction is carried out in the presence of triethylamine, of Na₂CO₃ or of K₂CO₃ in acetonitrile.

10. Process according to any one of Claims 1 to 9, **characterized in that** the reaction is carried out in the presence of Na₂CO₃ in acetonitrile heated at reflux.

11. Process according to Claim 1 for the preparation of N-piperidino-5-(4-chlorophenyl)-1-(2,4-dichlorophenyl)-4-methylpyrazole-3-carboxamide and of its salts, **characterized in that** 1,5-dibromopentane is reacted with a compound of formula: 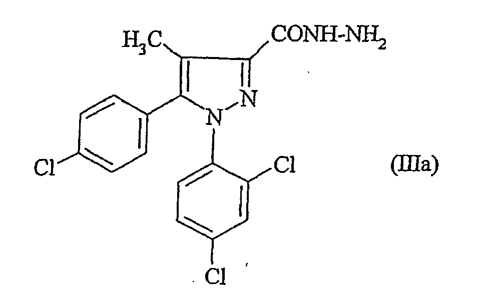 in the presence of Na₂CO₃ in acetonitrile heated at reflux.

## Patentansprüche

1. Verfahren zur Herstellung einer Verbindung der Formel: 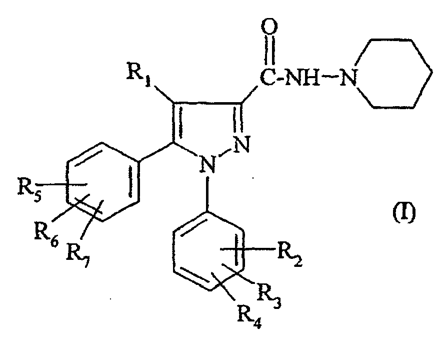 worin:
- R₁ für ein Wasserstoff- oder Halogenatom oder eine (C₁-C₄) -Alkylgruppe steht;
- R₂ , R₃ , R₄ , R₅, R₆ und R₇ jeweils unabhängig voneinander für ein Wasserstoff- oder Halogenatom oder eine (C₁-C₄) -Alkyl-, (C₁-C₄) -Alkoxy- oder Trifluormethylgruppe stehen;
und Salzen davon, **dadurch gekennzeichnet, daß**
man ein Pentanderivat der Formel:
X- (CH₂) ₅-X' (I)
worin X und X' jeweils unabhängig voneinander für ein Wasserstoffatom oder eine YSO₂-Gruppe, worin Y für eine (C₁-C₄) -Alkylgruppe, eine (C₁-C₄) -Perfluoralkylgruppe oder eine Phenylgruppe, die gegebenenfalls durch eine Methyl-, Chlor- oder Nitrogruppe substituiert ist, steht, stehen, in Gegenwart einer Base in einem Lösungsmittel und bei einer Temperatur zwischen Umgebungstemperatur und der Rückflußtemperatur des Lösungsmittels mit einem Pyrazol-3-carbohydrazidderivat der Formel: 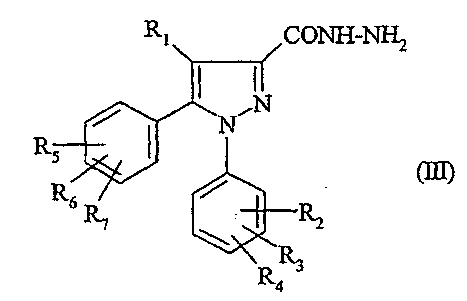 worin R₁, R₂, R₃, R₄, R₅, R₆ und R₇ wie oben für (I) definiert sind, umsetzt.

2. Verfahren nach Anspruch 1, **dadurch gekennzeichnet, daß** in der Verbindung der Formel (II) X und X' jeweils unabhängig voneinander für ein Halogenatom stehen.

3. Verfahren nach Anspruch 1, **dadurch gekennzeichnet, daß** in der Verbindung der Formel (II) X und X' jeweils unabhängig voneinander für eine Y-SO₂-O-Gruppe, worin Y wie in Anspruch 1 definiert ist, stehen.

4. Verfahren nach einem der Ansprüche 1 bis 3 zur Herstellung von N-Piperidino-5-(4-chlorphenyl)-1-(2,4-dichlorphenyl)-4-methylpyrazol-3-carboxamid und Salzen davon, **dadurch gekennzeichnet, daß** man eine Verbindung der Formel (II) mit 5-(4-Chlorphenyl)-1-(2,4-dichlorphenyl)-4-methyl-1*H-*pyrazol-3-carbohydrazid der Formel: 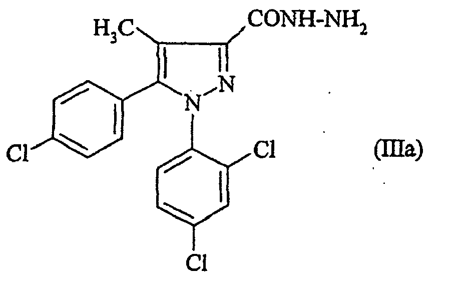 umsetzt.

5. Verfahren nach Anspruch 4, **dadurch gekennzeichnet, daß** man ein 1,5-Dihalogenpentan mit der Verbindung der Formel (IIIa) umsetzt.

6. Verfahren nach Anspruch 5, **dadurch gekennzeichnet, daß** man ein 1,5-Dibrompentan mit der Verbindung der Formel (IIIa) umsetzt.

7. Verfahren nach einem der Ansprüche 1 bis 6,
**dadurch gekennzeichnet, daß** man die Base unter Triethylamin, NaOH; KOH, K₂CO₃, Na₂CO₃ und Cs₂CO₃ auswählt.

8. Verfahren nach einem der Ansprüche 1 bis 7,
**dadurch gekennzeichnet, daß** man das Lösungsmittel unter Toluol, Chlorbenzol, Tetrahydrofuran, Dimethoxyethan, Dioxan, Acetonitril oder Propionitril auswählt.

9. Verfahren nach einem der Ansprüche 1 bis 8,
**dadurch gekennzeichnet, daß** man die Umsetzung in Gegenwart von Triethylamin, Na₂CO₃ oder K₂CO₃ in Acetonitril durchführt.

10. Verfahren nach einem der Ansprüche 1 bis 9,
**dadurch gekennzeichnet, daß** man die Umsetzung in Gegenwart von Na₂CO₃ in zum Rückfluß erhitztem Acetonitril durchführt.

11. Verfahren nach Anspruch 1 zur Herstellung von N-Piperidino-5-(4-chlorphenyl)-1-(2,4-dichlorphenyl)-4-methylpyrazol-3-carboxamid und Salzen davon, **dadurch gekennzeichnet, daß** man 1,5-Dibrompentan in Gegenwart von Na₂CO₃ in zum Rückfluß erhitztem Acetonitril mit einer Verbindung der Formel:
